Europäisches Patentamt

European Patent Office

Office européen des brevets

(19)

(11) **EP 1 473 045 A1**

(12) **EUROPEAN PATENT APPLICATION**
published in accordance with Art. 158(3) EPC

(43) Date of publication:
**03.11.2004 Bulletin 2004/45**

(21) Application number: **03734628.5**

(22) Date of filing: **30.01.2003**

(51) Int Cl.⁷: **A61L 9/014**, A61L 9/16, B01J 20/12, B01D 53/44

(86) International application number:
**PCT/JP2003/000886**

(87) International publication number:
**WO 2003/063921 (07.08.2003 Gazette 2003/32)**

(84) Designated Contracting States:
**AT BE BG CH CY CZ DE DK EE ES FI FR GB GR HU IE IT LI LU MC NL PT SE SI SK TR**

(30) Priority: **31.01.2002 JP 2002023918**

(71) Applicant: **Midori Anzen Co., Ltd.
Tokyo 150-8455 (JP)**

(72) Inventors:
• **MIFUNE, Yuzo, MIDORI ANZEN CO., LTD
Tokyo 150-8455 (JP)**
• **WATANABE, Katsuomi,
MIDORI ANZEN CO., LTD
Tokyo 150-8455 (JP)**

(74) Representative: **Strehl Schübel-Hopf & Partner
Maximilianstrasse 54
80538 München (DE)**

(54) **DEODORANT MATERIAL AND PROCESS FOR PRODUCING THE SAME**

(57)     The invention provides a deodorizing article which can be used semi-permanently and maintains sufficient mechanical strength under usual conditions of use and a method for producing the deodorizing article.

The deodorizing article of the invention is produced by dispersing in a medium a clay mineral having mesopores, gypsum series, a mineral having a pore size greater than that of the gypsum series, and a white inorganic filler, and molding the formed dispersion.

FIG.1

EP 1 473 045 A1

**Description**

Technical Field

The present invention relates to a deodorizing article which can be used semi-permanently, and more particularly to a deodorizing article which effectively removes an oil-related odor generated from a kitchen. The invention also relates to a method for producing the deodorizing article.

Background Art

Conventionally, there have been known methods for removing offensive odor substances from a variety of sources of offensive odor; e.g., a deodorization method by adsorption ; a chemical deodorization method including neutralization and decomposition of an odorous substance by use of a chemical; and a biological deodorization method including decomposition of an odor component by use of a microorganism.

Generally, the adsorption deodorization method employs activated carbon. After the activated carbon has been used for a certain period of time, the effect of activated carbon weakens considerably to such a level that substantially no deodorization is expected. Therefore, the deodorizing material must be replaced by new material at predetermined intervals, which is cumbersome.

A problem arises upon employment of the chemical deodorization method. Specifically, the method requires direct contact between an odorous substance and a chemical for causing chemical reaction. Thus, such a chemical requires additional care such as periodical replacement or replenishment, and handling of chemicals involves hazard. This situation is also problematic.

The biological deodorization method is less hazardous. However, the method also involves problems such as requiring a large deodorization facility and a water-supply facility.

These conventional methods require maintenance work such as replacement/replenishment of material or supply of water. In the event of failure to perform the maintenance, the article and method employed for deodorization fail to function. In other words, a offensive odor cannot be removed even though the deodorizing article is employed in combination with the deodorizing method.

In order to solve the aforementioned problems, the present applicant previously filed an application (Japanese Patent Application Laid-Open (*kokai*) No. 2002-095730) directed to a semi-permanently usable deodorizing article developed by the applicant. The previous invention had been accomplished on the basis of the inventors' finding that a deodorizing article formed by dispersing in gypsum series a clay mineral having a tunnel-like micro-structure (e.g., sepiolite or palygorskite), which mineral generally has one-dimensional tunnel pores (i.e., micro-pores) and meso-pores, exerts a semi-permanent deodorization effect on the basis of adsorption and desorption of an odor substance, in cooperation with the moisture-maintenance effect of the gypsum series. More specifically, in the city of Tokyo, the odor concentration of exhaust from kitchens of restaurants and other food businesses is restricted to fall under a regulation standard in accordance with the Tokyo Metropolitan Environmental Pollution Ordinance. Under these circumstances, although pursuing a deodorizing article which attains complete deodorization may have some meaning, from a viewpoint that takes into consideration use of the article involving the aforementioned maintenance, a deodorizing article which can suppress the odor concentration to a level below regulation standard determined by, for example, ordinances or to a level below a certain concentration (e.g., a low odor concentration where an offensive odor is not sensed) and which can be used semi-permanently could be more effective. The previous inventors have considered this viewpoint.

However, poor mechanical strength of the deodorizing article raises the problem that the deodorizing article is readily damaged (e.g., broken) during an actual installation operation.

Disclosure of the Invention

In view of the foregoing, an object of the present invention is to provide a deodorizing article which can be used semi-permanently and maintains sufficient mechanical strength under usual conditions of use. Another object of the invention is to provide a method for producing the deodorizing article.

In order to solve the aforementioned problems, a first mode of the present invention is drawn to a deodorizing article, characterized in that the deodorizing article is produced by dispersing in a medium a clay mineral having meso-pores, gypsum series, a mineral having a pore size greater than that of the gypsum series, and a white inorganic filler, and molding the formed dispersion.

According to the first mode, mechanical strength of the article is enhanced by the white inorganic filler while a semi-permanent deodorization effect is maintained, the effect being exerted by the meso-pores of the clay mineral on the basis of adsorption and desorption of an odor substance, in cooperation with the moisture-maintenance effect of the gypsum series.

A second mode of the present invention is drawn to a specific embodiment of the deodorizing article of the first mode, which further contains a finely divided powder of a clay mineral having meso-pores.

According to the second mode, a state in which each raw material is uniformly dispersed is consistently attained, thereby producing deodorizing articles having no variation in performance.

A third mode of the present invention is drawn to a specific embodiment of the deodorizing article of the first or second mode, which has a bending strength of 0.49 MPa or higher.

According to the third mode, the deodorizing article has such a mechanical strength that will not permit collapsing of the article during manual handling.

A fourth mode of the present invention is drawn to a specific embodiment of the deodorizing article of any of the first to third modes, wherein the clay mineral is at least one species selected from sepiolite and palygorskite, and the mineral having a pore size greater than that of the gypsum series is expanded perlite.

According to the fourth mode, the meso-pores of sepiolite or palygorskite exert a semi-permanent deodorization effect on the basis of adsorption and desorption of an odor substance, in cooperation with the moisture-maintenance effect of the gypsum series, and desorption of substances which have been adsorbed by the clay mineral is promoted by the presence of macro-pores of expanded perlite.

A fifth mode of the present invention is drawn to a specific embodiment of the deodorizing article of any of the first to fourth modes, which contains the clay mineral in an amount of at least 30 wt.% as solid content, the gypsum series in an amount of at least 25 wt.% as solid content, and the mineral having a pore size greater than that of the gypsum series in an amount of 30 wt.% or less as solid content.

According to the fifth mode, the form of the deodorizing article is satisfactorily maintained, an excellent balance can be attained between adsorption and desorption, and mechanical strength is enhanced.

A sixth mode of the present invention is drawn to a specific embodiment of the deodorizing article of any of the first to fifth modes, wherein the white inorganic filler is at least one species selected from colloidal silica and cement.

According to the sixth mode, mechanical strength is enhanced by colloidal silica or cement, without lowering the semi-permanent deodorization effect on the basis of adsorption and desorption of an odor substance.

A seventh mode of the present invention is drawn to a specific embodiment of the deodorizing article of the sixth mode, wherein the white inorganic filler is colloidal silica and is contained in an amount of 10 to 20 wt.% as solid content.

According to the seventh mode, mechanical strength is effectively enhanced by colloidal silica, without lowering the semi-permanent deodorization effect on the basis of adsorption and desorption of an odor substance.

An eighth mode of the present invention is drawn to a specific embodiment of the deodorizing article of the sixth mode, wherein the white inorganic filler is cement and is contained in an amount of 14 to 37 wt.% as solid content.

According to the eighth mode, mechanical strength is effectively enhanced by cement, without lowering the semi-permanent deodorization effect on the basis of adsorption and desorption of an odor substance.

A ninth mode of the present invention is drawn to a specific embodiment of the deodorizing article of any of the first to eighth modes, which is molded through slip cast molding.

According to the ninth mode, a favorable dispersion state of the clay mineral in the gypsum series is maintained during slip cast molding, and the mechanical strength required for usual use can be ensured.

A tenth mode of the present invention is drawn to a specific embodiment of the deodorizing article of any of the first to ninth modes, which is, in use, provided in an exhaust system connected to a kitchen.

According to the tenth mode, an offensive odor such as an oil-related odor generated in a kitchen is reduced to a certain level or lower.

An eleventh mode of the present invention is drawn to a specific embodiment of the deodorizing article of the tenth mode, which has a hollow cylindrical or columnar form and has a plurality of flow lines penetrated in an axial direction.

According to the eleventh mode, the deodorizing article has such a mechanical strength that will not permit collapsing of the article during manual handling upon operations such as installation of the article in a duct line.

A twelfth mode of the present invention is directed to a method for producing a deodorizing article, characterized in that the method comprises the steps of dispersing in water a clay mineral having meso-pores, gypsum series, a mineral having a pore size greater than that of the gypsum series, and a white inorganic filler, to thereby form a dispersion; charging the dispersion into a mold through slip casting, and drying the dispersion and releasing the dried product from the mold.

According to the twelfth mode, a deodorizing article having a mechanical strength enhanced by the white inorganic filler is produced while a semi-permanent deodorization effect is maintained, the effect being exerted by the meso-pores of the clay mineral on the basis of adsorption and desorption of an odor substance, in cooperation with the moisture-maintenance effect of the gypsum series.

A thirteenth mode of the present invention is drawn to a specific embodiment of the method for producing a deodorizing article of the twelfth mode, wherein the step of dispersing the members in water to thereby form a dispersion further includes dispersing a finely divided powder of a clay mineral having meso-pores.

According to the thirteenth mode, a state in which each raw material is uniformly dispersed is consistently attained, thereby producing desired deodorizing articles having no variation in performance.

A fourteenth mode of the present invention is drawn to a specific embodiment of the method for producing a deodorizing article of the twelfth or thirteenth mode, which further includes a step of preliminary firing at a predetermined temperature the clay mineral having meso-pores.

According to the fourteenth mode, the clay mineral that has been subjected to preliminary firing is dispersed in the gypsum series, and the resultant mixture is molded. Thus, the meso-pores of the clay mineral function effectively, to thereby provide a deodorizing article exhibiting excellent deodorization performance and mechanical strength.

A fifteenth mode of the present invention is drawn to a specific embodiment of the method for producing a deodorizing article of any of the twelfth to fourteenth modes, wherein the clay mineral is at least one species selected from sepiolite and palygorskite, and the mineral having a pore size greater than that of the gypsum series is expanded perlite.

According to the fifteenth mode, there is produced a deodorizing article which exhibits an excellent balance between adsorption and desorption maintained by sepiolite or palygorskite and perlite, and which has satisfactory mechanical strength.

A sixteenth mode of the present invention is drawn to a specific embodiment of the method for producing a deodorizing article of any of the twelfth to fifteenth modes, wherein the deodorizing article contains the clay mineral in an amount of at least 30 wt.% as solid content, the gypsum series in an amount of at least 25 wt.% as solid content, and the mineral having a pore size greater than that of the gypsum series in an amount of 30 wt.% or less as solid content.

According to the sixteenth mode, there is produced a deodorizing article which exhibits an excellent balance between adsorption and desorption and satisfactory mechanical strength, and the form of the deodorizing article is satisfactorily maintained.

A seventeenth mode of the present invention is drawn to a specific embodiment of the method for producing a deodorizing article of any of the twelfth to sixteenth modes, wherein the white inorganic filler is at least one species selected from colloidal silica and cement.

According to the seventeenth mode, mechanical strength is enhanced by colloidal silica or cement, without lowering the semi-permanent deodorization effect on the basis of adsorption and desorption of an odor substance.

An eighteenth mode of the present invention is drawn to a specific embodiment of the method for producing a deodorizing article of the seventeenth mode, wherein the white inorganic filler is colloidal silica and is contained in an amount of 10 to 20 wt.% as solid content.

According to the eighteenth mode, mechanical strength is effectively enhanced by colloidal silica, without lowering the semi-permanent deodorization effect on the basis of adsorption and desorption of an odor substance.

A nineteenth mode of the present invention is drawn to a specific embodiment of the method for producing a deodorizing article of the seventeenth mode, wherein the white inorganic filler is cement and is contained in an amount of 14 to 37 wt.% as solid content.

According to the nineteenth mode, mechanical strength is effectively enhanced by cement, without lowering the semi-permanent deodorization effect on the basis of adsorption and desorption of an odor substance.

The present invention will next be described in detail.

Examples of the clay mineral having meso-pores which may be employed in the present invention include clay minerals having a tunnel-like micro-structure such as sepiolite and palygorskite, and synthetic clay minerals such as meso-porous materials (e.g., meso-porous silica and meso-porous molecular sieve), silica gel, aluminum oxide (alumina), and carbon aerogel. For example, sepiolite has one-dimensional tunnel pores (i.e., micro-pores) and meso-pores, and more specifically, has micro-pores of 0.5 to 1.1 nm originating from the crystal structure thereof, and meso-pores of some tens of nm corresponding to interparticle spacing.

Such clay minerals are generally known to exert moisture-controlling effect. According to the present invention, a semi-permanent deodorization effect on the basis of adsorption and desorption of an odor substance is exerted in cooperation with the moisture-maintenance effect of the gypsum series.

No particular limitation is imposed on the particle size of the clay mineral. The particle is generally about 0.2 to about 2 mm.

The gypsum series which can be employed in the present invention is a compound which comprises calcium sulfate as a predominant component and can be molded into a desired shape through reaction with water. Either naturally occurring gypsums or chemically synthesized gypsums may be employed. Examples of the gypsums include crystalline gypsum, hemihydrate, anhydrous gypsum, and plaster of Paris. Generally, commercial plaster of Paris (calcined gypsum) is used.

In the present invention, the clay mineral having meso-pores is preferably used in an amount of at least 30 wt.% as solid content in order to attain excellent adsorption performance.

The gypsum series must be used in an amount of at least 25 wt.% as solid content for maintaining the shape of the deodorizing article. Thus, when the deodorizing article is formed from two components, the gypsum series content is preferably 25 to 70 wt.% as solid content.

The aforementioned gypsum series maintains the shape of the deodorizing article of the present invention and serves as a water-supply material. Thus, the gypsum series preferably comprises a material which provides macropores of 1 to 10 μm, preferably about 5 μm, after molding thereof.

The deodorizing article of the present invention contains gypsum which surrounds sepiolite having micropores and meso-pores. The gypsum, having macro-pores of 1 to 10 μm, serves as a water-supply material. An adsorption-desorption mechanism involving sepiolite and gypsum is considered as follows. When an atmosphere or air which passes through the deodorizing article contains an odor component at high level, sepiolite adsorbs the odor component which has passed through pores of gypsum, and in turn transfers water which has been adsorbed by sepiolite to gypsum, to thereby lower the odor level to a certain level or lower.

Even when the odor component does not exist, gypsum continues to adsorb water until adsorption of water reaches the saturation state. Sepiolite, which exerts moisture-controlling effect, adsorbs water contained therein. The odor component which has been adsorbed by sepiolite is gradually released through substitution; i.e., competitive adsorption, to thereby lower the odor level to a certain level or lower (i.e., desorption).

On the basis of the function of water, the deodorizing article of the present invention exerts particularly remarkable effect of removing an oil-related odor which is generated in a place such as a kitchen where vaporization of water is prone to occur during the course of cooking.

As described above, by virtue of the synergistic effect exerted by gypsum series and a clay mineral such as sepiolite, the deodorizing article of the present invention adsorbs an odor substance to thereby lower the odor level to a certain level or lower, when the target gas contains a large amount of an odor component, and releases the odor component without elevating the level of the released odor component to a certain level or higher, when the odor component level is lowered. By repeating the procedure, the odor level can be averaged and lowered to a certain level or lower, with the level being suppressed semi-permanently.

The deodorizing article of the present invention preferably contains a mineral having a pore size greater than that of gypsum series, in view of promotion of the aforementioned desorption of the odor substance.

Examples of minerals having a pore size greater than that of gypsum series include expanded perlite (may be referred to as foamed perlite). The expanded perlite, which *per se* has no adsorption ability, increases the entire volume of a mixture of gypsum series and a clay mineral having meso-pores when incorporated into the mixture. Since the macro-pore size of the additional mineral is greater than that of gypsum series, the aforementioned adsorption-desorption is considered to be promoted.

The amount of the mineral having a large pore size is controlled so as not lower the relative amounts of active ingredients and so as not to greatly lower overall mechanical strength. Thus, the amount is controlled to 30 wt.% or less, preferably 8 to 12.5 wt.% as solid content. Although the additional mineral is optional, the mineral is preferably added in an amount of at least 8 wt.% in order to fully attain the effect thereof.

No particular limitation is imposed on the particle size of expanded perlite, and the particle size is generally 0.15 to 1 mm.

It is preferred that the deodorizing article of the present invention contains a white inorganic filler in order to enhance mechanical strength. The white inorganic filler employed herein is an inorganic filler such as colloidal silica or cement and is capable of enhancing the mechanical strength of the deodorizing article, without considerably lowering deodorizing performance. It is also preferred that the deodorizing article of the present invention is molded by dispersing raw materials in water to form a dispersion and molding the dispersion through slip casting. Thus, preferably, the raw materials enhance mechanical strength of the deodorizing article while favorable moldability of the dispersion containing the raw materials is maintained.

From the above viewpoint, the white inorganic filler is preferably selected from colloidal silica and cement, in consideration of viscosity and pot life of a dispersion containing the filler, enhancement of mechanical strength, retention of deodorization performance, or other factors.

The amount of the white inorganic filler may be determined in consideration of the aforementioned factors. In general, the amount, which varies depending on the type of the filler employed, is preferably about 10 to 20 wt.% as solid content in the case of colloidal silica, and preferably about 14 to 37 wt.% as solid content in the case of cement. When the amount is less than the lower limit of the above ranges, enhancement of mechanical strength is insufficiently attained, whereas when the amount exceeds the above ranges, deodorization performance becomes poor or handling of a dispersion during molding is considerably impaired.

The deodorizing article of the present invention preferably contains a finely divided particle of a clay mineral. The finely divided powder is produced from a clay mineral having meso-pores, preferably having a particle size of 75 μm or less, more preferably 45 μm or less. Examples of such a finely divided powder of a clay mineral include finely divided sepiolite powder (trade name: Milcon SP-2, product of Showa Mining Co., Ltd.).

When gypsum series, a clay mineral, a mineral having a pore size greater than that of the gypsum series, and a finely divided powder of a clay mineral are dispersed in water and the dispersion is poured into a mold through slip casting, there can be prevented precipitation of the clay mineral and floatation of the mineral having a pore size greater than that of the gypsum series, before completion of curing of the gypsum series. Thus, when a mixture of raw materials contains a finely divided powder of a clay mineral, a state in which each raw material is uniformly dispersed is consistently attained during a curing period. Such a stable dispersion state is particularly effective for molding by use of a mold having a long side length. Accordingly, there can be produced desired deodorizing articles in which each raw material is uniformly dispersed and which have less variation in performance.

The finely divided powder of a clay mineral exerting the above effect is preferably contained in an amount of about 0.5 to 3.0 wt.%. When the amount is less than the lower limit, the effect is insufficiently attained, whereas when the amount in excess of the upper limit, deodorization performance is lowered.

Although some clay minerals exert the same effect, use of a finely divided powder of a clay mineral having meso-pores, serving as a predominant component of the mixture of raw materials, is preferred so as not to considerably impair, through addition of the clay mineral, deodorization characteristics, *inter alia,* the desorption effect upon repeated use of the deodorization article.

The deodorizing article of the present invention is produced by mixing raw materials with dispersion, charging the resultant mixture into a mold, and drying the mixture.

No particular limitation is imposed on the shape, dimensions, etc. of the deodorizing article. However, since the deodorization is effected on the basis of adsorption (contact with the odor substance), the deodorizing article preferably has a structure assuring a large contact area. For example, a columnar article or a prismatic article of a honeycomb structure having flow lines penetrated in an axial direction is preferred. A typical hollow cylinder, prism, etc. may also be employed.

No particular limitation is imposed on the method for producing the deodorizing article of the present invention. After completion of mixing and dispersing raw materials, molding the resultant mixture by drying can be performed under the same conditions as employed for yielding typical gypsum series products.

However, as mentioned in the Test Examples below, preliminary firing of sepiolite is preferably performed before mixing and dispersing of raw materials. Preliminary firing is considered to prevent crushing or similar trouble of sepiolite during mixing and dispersion of raw materials, thereby enhancing desorption performance of the molded deodorizing article. No particular limitation is imposed on the firing conditions, so long as the aforementioned effect is attained. For example, firing is performed at 400 to 800°C for about one hour.

According to the deodorizing article of the present invention, bending strength of the article is enhanced to 0.49 MPa (5 kg/cm$^2$) or higher by the effect of the white inorganic filler while a semi-permanent deodorization effect is maintained, the effect being exerted by the meso-pores of the clay mineral on the basis of repeated adsorption and desorption of an odor substance, in cooperation with the moisture-maintenance effect of the gypsum series. Thus, the deodorizing article has such a mechanical strength that will not permit collapsing of the article during ordinary handling such as manual handling.

When a microorganism is caused to adhere to the deodorizing article (the microorganism being not intentionally caused to adhere during use of the deodorizing article of the present invention), the microorganism ingests, as nutrient, an odor component adsorbed by the deodorizing article. In addition, the microorganism produces an enzyme under the conditions satisfying requirements for water content, temperature, etc.

By virtue of enzymatic reaction, an accumulated odor component which has been adsorbed but not desorbed is decomposed, to thereby prevent clogging or similar trouble and renew adsorption effect, leading to further semi-permanent deodorization performance. Such performance regeneration effect due to a microorganism is attributed to a microorganism present in the atmosphere. Thus, the effect is generally exerted when the deodorizing article is used in the atmosphere. Particularly when the article is used in a kitchen for removing an oil-related odor, an oil-decomposing enzyme such as lipase or lipase-producing bacteria *per se* may be caused to adhere to the article in advance.

Brief Description of the Drawings

Fig. 1 is a schematic perspective view of the deodorizing article according to one embodiment of the present invention.

Fig. 2 is a graph showing the results of Test Example 3 carried out in relation to the present invention.

Fig. 3 is a graph showing the results of Test Example 4 carried out in relation to the present invention.

Fig. 4 shows the procedure of Test Example 6 carried out in relation to the present invention.

Fig. 5 is a graph showing the results of Test Example 6 carried out in relation to the present invention.

Fig. 6 is a graph showing the results of Test Example 7 carried out in relation to the present invention.

Fig. 7 is a graph showing the pore size distribution profile obtained in Test Example 8 carried out in relation to the present invention.

Fig. 8 is a graph showing the pore size distribution profile obtained in Test Example 8 carried out in relation to the present invention.

Fig. 9 is a graph showing the pore size distribution profile obtained in Test Example 8 carried out in relation to the present invention.

Fig. 10 is a graph showing the pore size distribution profile obtained in Test Example 8 carried out in relation to the present invention.

Best Mode for Carrying Out the Invention

The present invention will next be described in detail by way of examples.

<Example 1>

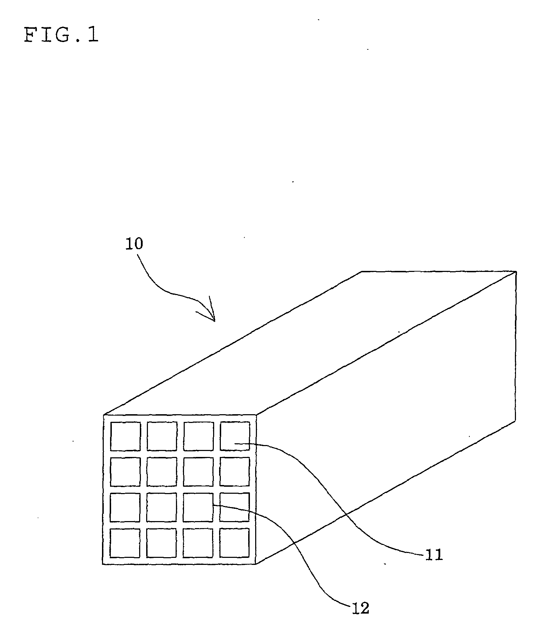

Sepiolite (particle size: 0.5 to 1.0 mm, naturally occurring in Turkey) was subjected to preliminary firing at 600°C for one hour. The thus-fired sepiolite, expanded perlite (for example, trade name: Topco Perlite), finely divided sepiolite powder (trade name: Milcon SP-2, product of Showa Mining Co., Ltd., the <45 μm-particle content: 99.6%), grade-B calcined gypsum, and a white inorganic filler (colloidal silica, Silicadol 20A (solid content 20%), product of Nippon Chemical Industrial Co., Ltd.) were blended in compositional proportions (parts by weight) shown in Table 1 together (each quantity in parentheses denotes a solid content (%); the same convention applies hereafter). Water was added to the mixture in an amount shown in Table 1. The mixed product was charged in a mold of a predetermined shape, and dried at 60°C for 12 hours, to thereby yield a 16-passage deodorizing article 10 as shown in Fig. 1. The deodorizing article 10 had outer dimensions of 10 cm × 10 cm × 20 cm, and 16 penetrated flow lines 11. Each penetrated flow line 11 was tapered, in consideration of releasing from the mold, and had one opening (18.7 × 18.7 mm) having a thickness of a partition wall 12 of 4.8 mm and the other opening (17.8 × 17.8 mm) having a partition wall thickness of 5.6 mm.

<Example 2>

The procedure of Example 1 was repeated, except that alumina cement was employed as a white inorganic filler instead of colloidal silica in an amount shown in Table 1, to thereby produce a deodorizing article.

<Example 3>

The procedure of Example 1 was repeated, except that portland cement was employed as a white inorganic filler instead of colloidal silica, in an amount shown in Table 1, to thereby produce a deodorizing article.

<Example 4>

The procedure of Example 1 was repeated, except that jet cement (Regulated Set Cement; ultra rapid-hardening cement) was employed as a white inorganic filler instead of colloidal silica, in an amount shown in Table 1, to thereby produce a deodorizing article.

<Comparative Example 1>

The procedure of Example 1 was repeated, except that compositional proportions shown in Table 1 were employed but no white inorganic filler was employed, to thereby produce a deodorizing article of Comparative Example 1.

<Comparative Example 2>

The procedure of Example 1 was repeated, except that carbon fiber (product of Mitsubishi Rayon Co., Ltd.) was employed instead of a white inorganic filler, in an amount shown in Table 1, to thereby produce a deodorizing article of

Comparative Example 2.

<Test Example 1>

In each Example and each Comparative Example, slip casting performance of a dispersion into a mold, pot life, and time required for removal from the mold were determined so as to evaluate handling efficiency. The results are shown in Table 1.

<Test Example 2>

In each Example and each Comparative Example, test samples having dimensions of 35 mm (width) $\times$ 70 mm (length) $\times$ 5 mm (thickness) were prepared. Bending strength (MPa) of each test sample was determined by use of a push-pull scale (product of Imada Co., Ltd.) in accordance with JIS K 6911 and the following equation. The results are shown in Table 1, along with molding properties and strength determined in Test Example 1 and overall evaluation. Overall evaluation is rated by O (passed both Test Example 1 and 2) and X (failed to pass at least one of these).

$$\text{Bending strength (MPa)} = 3PL/2Wt^2$$

P: Load (N) to cause deflection of a test sample
L: Distance between supports (mm)
W: Width of a sample (mm)
t: Thickness of a sample (mm)

The results of Test Examples 1 and 2 revealed that test samples of Examples 1 to 4 exhibited molding properties; i.e., handling efficiency nearly equal to that of the test sample of Comparative Example 1 containing no white inorganic filler, and a mechanical strength; i.e., a bending strength was enhanced to a level higher than 0.49 MPa. The test results confirmed that mechanical strength was not remarkably enhanced in the sample of Comparative Example 2 containing carbon fiber as a reinforcement material, and handling efficiency decreased. Use of glass fiber and use of other inorganic fibers were also confirmed to. fail to improve mechanical strength and handling efficiency.

[Table 1]

| | | Ex. 1 | Ex. 2 | Ex. 3 | Ex. 4 | Comp. Ex. 1 | Comp. Ex. 2 |
|---|---|---|---|---|---|---|---|
| Raw materials parts by weight | Sepiolite | 20 (39.4%) | 20 (35.2%) | 20 (35.2%) | 20 (35.2%) | 20 (49.0%) | 20 (48.8%) |
| | Perlite | 4 (7.9%) | 4 (7.0%) | 4 (7.0%) | 4 (7.0%) | 4 (9.8%) | 4 (9.8%) |
| | Sepiolite fine powder | 0.8 (1.6%) | 0.8 (1.4%) | 0.8 (1.4%) | 0.8 (1.4%) | 0.8 (2.0%) | 0.8 (2.0%) |
| | Gypsum series | 16 (31.5%) | 16 (28.2%) | 16 (28.2%) | 16 (28.2%) | 16 (39.2%) | 16 (39.0%) |
| | Colloidal silica | 50 (19.7%) | — | — | — | — | — |
| | Cement | — | 16 (28.2%) | 16 (28.2%) | 16 (28.2%) | — | — |
| | Water | 11.8 | 52 | 52 | 52 | 52 | 52 |
| | Carbon fiber | — | — | — | — | — | 0.2 (0.5%) |
| Slip casting to mold | | good*1 | good | good | good | good | good*1 |
| Pot life (min) | | ≧5 | ≧15 | ≧5 | ≧15 | ≧15 | ≧15 |
| Release time (min) | | ≧45 | ≧45 | ≧45 | ≧45 | ≧45 | ≧45 |
| Bending strength (MPa) | | 1.26 | 0.80 | 0.69 | 0.74 | 0.23 | 0.35 |
| Overall evaluation | | O | O | O | O | X | X |

Amount (parts by weight) of raw material in the parentheses represents a solid content.

*1: Viscosity slightly higher than that of Comp. Ex. 1

<Examples 5a to 5d>

The procedure of Example 1 was repeated, except that the amount of colloidal silica was altered to 12.5 parts by weight, 25 parts by weight, 50 parts by weight, or 75 parts by weight (Examples 5a to 5d) as shown in Table 2, to thereby produce a deodorizing article.

<Test Example 3>

In a manner similar to that of Test Example 1 or 2, a slip casting performance of a dispersion into a mold, pot life, time required for removal from the mold, and bending strength in relation to Examples 5a to 5d were determined. The results are shown in Table 2.

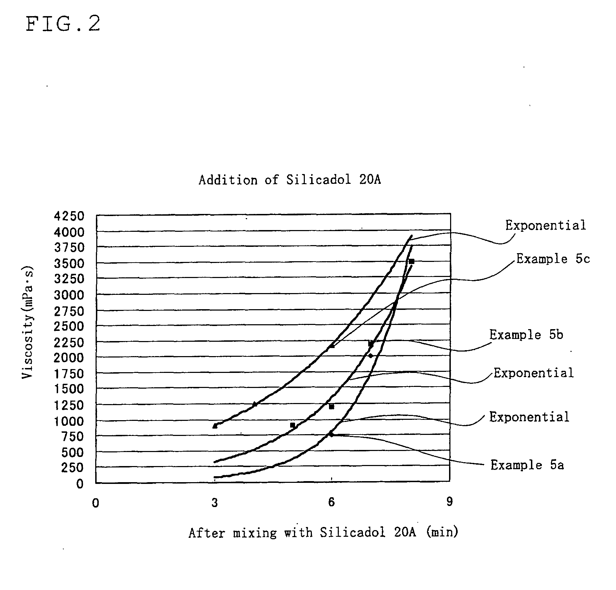

In Examples 5a to 5c, the time-dependent change of viscosity (mPa·s) of a dispersion after completion of mixing of raw materials was measured. The results are shown in Fig. 2.

The results indicate the following. During slip cast molding, the dispersion preferably has a viscosity of 750 to 1,750 mPa·s, and the viscosity is preferably maintained within the range for a specific period of time. When the viscosity of the produced dispersion remains less than 750 mPa·s for a long time, components contained in the dispersion are settled, in a sequence corresponding to specific gravity, before or after slip casting into a mold, yielding a deodorizing article of unsatisfactory performance. When the viscosity of the produced dispersion surpasses 1,750 mPa·s in a short period of time, the dispersion entrains air or is poorly charged, due to low mobility during slip casting into a mold, imposing restrictions on the slip casting operation. Needless to say, both cases are not preferred. The dispersion of Example 5a, containing colloidal silica at a solid content of 5.8%, provides sufficiently enhanced bending strength. However, raw material components readily cause separation, and six minutes after preparation of the dispersion, the viscosity, which has an initial value of 750 mPa·s, drastically increases. Thus, the dispersion is not suited for practical use. The dispersion of Example 5d, containing colloidal silica at a solid content of 26.9%, causes gelation of raw materials, thereby impairing moldability. The dispersions of Examples 5b and 5c, containing colloidal silica at solid contents of 10.9% and 19.7%, respectively, provide excellent moldability and enhanced bending strength.

[Table 2]

| Raw materials parts by weight | | Ex. 5a | Ex. 5b | Ex. 5c | Ex. 5d |
|---|---|---|---|---|---|
| | Sepiolite | 20 (46.2%) | 20 (43.7%) | 20 (39.4%) | 20 (35.8%) |
| | Perlite | 4 (9.2%) | 4 (8.7%) | 4 (7.9%) | 4 (7.2%) |
| | Sepiolite fine powder | 0.8 (1.8%) | 0.8 (1.7%) | 0.8 (1.6%) | 0.8 (1.4%) |
| | Gypsum series | 16 (37.0%) | 16 (34.9%) | 16 (31.5%) | 16 (28.7%) |
| | Colloidal silica | 12.5 (5.8%) | 25 (10.9%) | 50 (19.7%) | 75 (26.9%) |
| | Water | 42 | 32 | 11.8 | 11.8 |
| Slip casting to mold | | separation | good*1 | good*1 | gelation |
| Pot life (min) | | ≅7 | ≅7 | ≅5 | ≅1 |
| Release time (min) | | ≅45 | ≅45 | ≅45 | ≅45 |
| Bending strength (MPa) | | 0.55 | 0.80 | 1.26 | 0.88 |
| Overall evaluation | | x | o | o | x |

Amount (parts by weight) of raw material in the parentheses represents a solid content.
*1: Viscosity slightly higher than that of Comp. Ex. 1

<Examples 6a to 6e>

The procedure of Example 2 was repeated, except that the amount of alumina cement was altered to 4 parts by weight, 8 parts by weight, 16 parts by weight, 24 parts by weight, or 32 parts by weight (Examples 6a to 6e), to thereby produce a deodorizing article.

<Test Example 4>

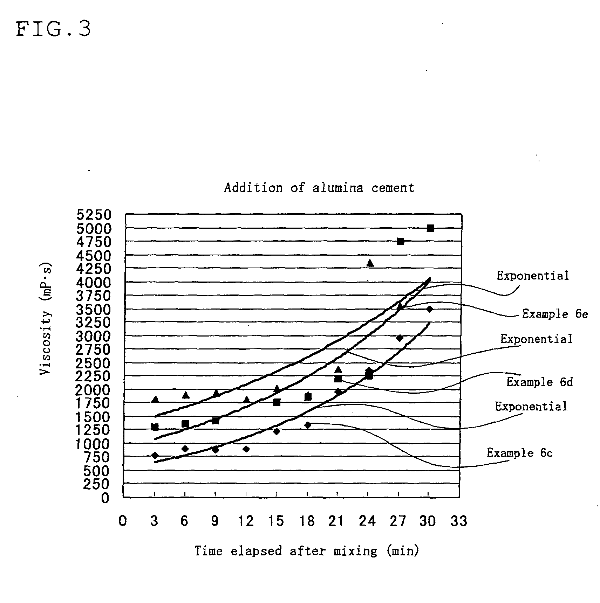

In a manner similar to that of Test Example 1 or 2, a slip casting performance of a dispersion into a mold, pot life, time required for removal from the mold, and bending strength in relation to Examples 6a to 6e were determined. The results are shown in Table 3.
In Examples 6c to 6e, the time-dependent change of viscosity (mPa·s) of a dispersion after completion of mixing of raw materials was measured. The results are shown in Fig. 3.
The results indicate the following. The dispersion of Example 6a, containing alumina cement at a solid content of 8.9%, provides insufficiently enhanced bending strength. The dispersion of Example 6e, containing alumina cement at a solid content of 44.0%, has high visicosity, resulting in poor moldability. The dispersions of Example 6b to 6d, containing alumina cement at solid contents of 16.4%, 28.2%, and 37.0%, respectively, provide excellent moldability and enhanced bending strength.

[Table 3]

| | | Ex. 6a | Ex. 6b | Ex. 6c | Ex. 6d | Ex. 6e |
|---|---|---|---|---|---|---|
| Raw materials parts by weight | Sepiolite | 20 (44.6%) | 20 (41.0%) | 20 (35.2%) | 20 (30.9%) | 20 (27.5%) |
| | Perlite | 4 (8.9%) | 4 (8.2%) | 4 (7.0%) | 4 (6.2%) | 4 (5.5%) |
| | Sepiolite fine powder | 0.8 (1.8%) | 0.8 (1.6%) | 0.8 (1.4%) | 0.8 (1.2%) | 0.8 (1.1%) |
| | Gypsum series | 16 (35.7%) | 16 (32.8%) | 16 (28.2%) | 16 (24.7%) | 16 (22.0%) |
| | Alumina cement | 4 (8.9%) | 8 (16.4%) | 16 (28.2%) | 24 (37.0%) | 32 (44.0%) |
| | Water | 52 | 52 | 52 | 52 | 52 |
| Slip casting to mold | | good | good | good | good | viscous |
| Pot life (min) | | ≧15 | ≧15 | ≧15 | ≧15 | ≧0 |
| Release time | | ≧45 | ≧45 | ≧45 | ≧45 | ≧45 |
| Bending strength (MPa) | | 0.37 | 0.52 | 0.80 | 0.82 | 0.89 |
| Overall evaluation | | X | O | O | O | X |

Amount (parts by weight) of raw material in the parentheses represents a solid content.

<Test Example 5>

The procedure of Example 2 was repeated, except that the amount of alumina cement was changed to 4, 5, 6, 7, 8, 16, or 32 parts by weight as shown in Table 4, to thereby produce a dispersion. The same test samples as those of Test Example 2 were molded from each dispersion and were subjected to bending test. The results are shown in Table 4.

The similar bending test was performed, except that portland cement was employed instead of alumina cement. The results are also shown in Table 4.

The results indicate that incorporation of either alumina cement or portland cement in an amount of 7 parts by weight (solid content: 14.6%) leads to a bending strength sufficient for install operation.

[Table 4]

| Formulation (parts by weight) | Bending strength (MPa) | |
|---|---|---|
| | Alumina cement | Portland cement |
| 4 | 0.37 | 0.39 |
| 5 | 0.44 | 0.45 |
| 6 | 0.47 | 0.47 |
| 7 | 0.50 | 0.54 |
| 8 | 0.52 | 0.59 |
| 16 | 0.80 | 0.69 |
| 32 | 0.89 | 0.70 |

<Test Example 6>

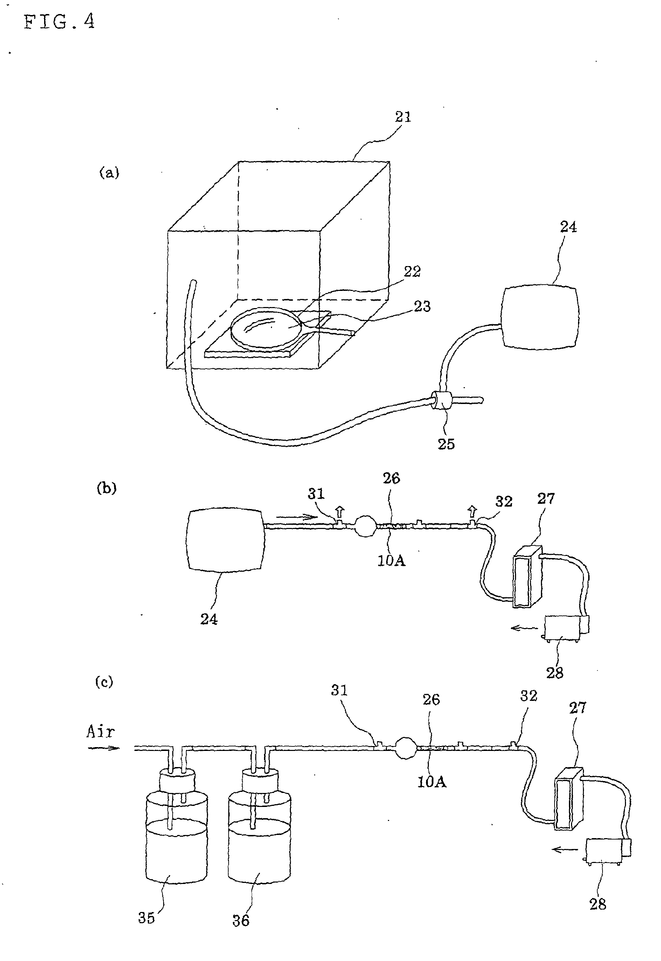

The deodorizing article of Example 6b was pulverized, to thereby provide test samples. Each sample was subjected to an oil-related gas adsorption and desorption repeating test in the following manner. Fig. 4 shows the procedure of the test.

(1) A sample obtained from the deodorizing article (see FIG. 1) was dried in an atmosphere at about 50°C for 24 hours (preliminary treatment), after which the dried sample was allowed to stand in a 50%-RH atmosphere at about 20°C for 24 hours.
(2) Oil 23 was added to a frying pan 22 placed in a chamber 21 and heated so as to generate oil-related gas. The gas present in the chamber 21 was sucked into a 20-L sampling bag 24 (hereinafter referred to simply as "bag") by means of a pump 25 (Fig. 4(a)).
(3) The gas concentration contained in the bag 24 was controlled to 20 to 25 ppm ($CH_4$) .
(4) A test sample 10A (2 g) was charged into a sample tube 26. One end of the sample tube was connected to the bag 24, and the other end of the sample tube was connected to a pump 28 via a flow meter 27.
(5) By operating the pump 28, the oil-related gas contained in the bag 24 was fed at 0.5 L/min into the sample tube 26. The gas was sampled every one minute for 31 minutes at an inlet sampling position 31 (upstream with respect to the sampling tube 26) and an outlet sampling position 32 (downstream with respect to the sampling tube 26) (Fig. 4(b)).
(6) Each gas sample was analyzed by means of a total hydrocarbon meter (detection limit: 0.1 ppm $CH_4$), and the amount of adsorbed gas component was calculated by integrating the difference in gas level between the inlet sampling potion and the outlet sampling position.
(7) The bag 24 was removed from the sample tube 26, and a silica gel trap 35 was connected to the sample tube 26 via an activated carbon trap 36. Air which had been passed through silica gel and activated carbon was fed to the sample tube 26 at 0.5 L/min for 15 minutes, to thereby effect desorption of the adsorbed gas component from the test sample 10A charged in the sample tube 26 (Fig. 4(c)).
(8) The sample tube 26 was removed and allowed to stand at 32°C for two days.
(9) The procedure including steps (2) to (8) was repeated.

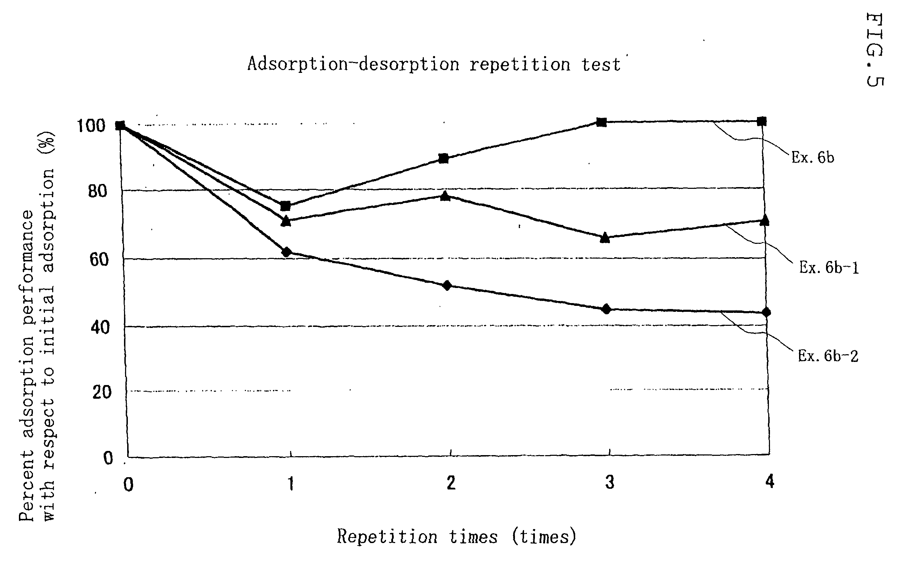

A test sample which had been subjected to the above procedure is denoted by "Ex. 6b sample" (microorganism-related). A test sample which had been subjected to a similar procedure, except that a membrane filter for removing microorganisms was provided on the upstream side of the test sample during desorption step (7), is denoted by "Ex. 6b-1 sample" (adsorption/desorption). A test sample which had been subjected to a similar procedure, except that the desorption step (7) was omitted, is denoted by "Ex. 6b-2 sample" (adsorption only).
The results are shown in Fig. 5. The results indicate that as compared with Ex. 6b sample, the adsorption effect of the deodorizing article (Ex. 6b-1 sample) decreases as the steps are performed repeatedly. The decrease in adsorption effect may be attributed to failure to attain microbial effect. Ex. 6b-2 sample which had not been subjected to any desorption step exhibited further decreased adsorption effect. Thus, these results confirm that the deodorizing article of the present invention releases an adsorbed odor component, and that the adsorption effect is renewed and maintained for a long period of time by virtue of microbial effect.

<Test Example 7>

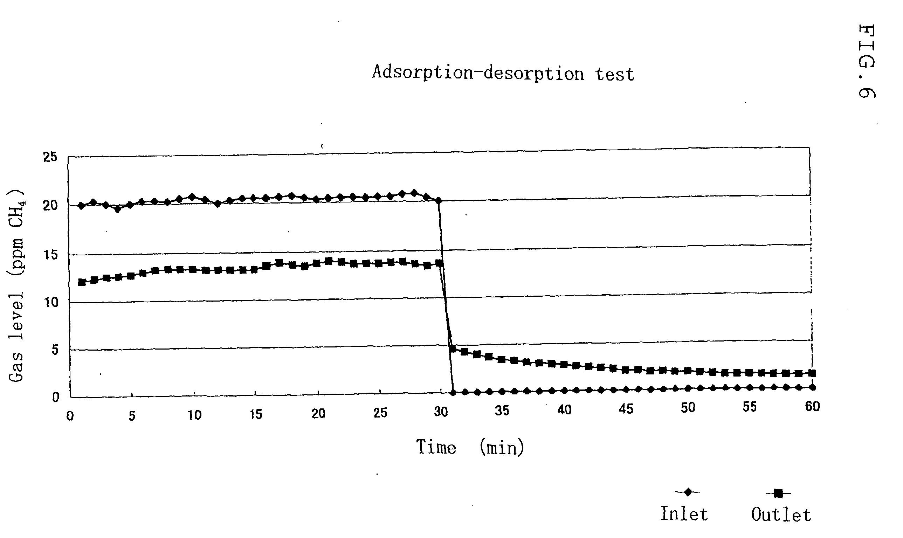

Test Example 7 was carried out in a manner similar to that of Test Example 6 employing the deodorizing article of Example 6b. After the adsorption step had been performed for 31 minutes, the outlet odor concentration of the gas was determined at every one minute while desorption was performed. The results are shown in Fig. 6.
The results indicate that the odor substance adsorbed over about 30 minutes was nearly completely released for 60 minutes.

<Test Example 8>

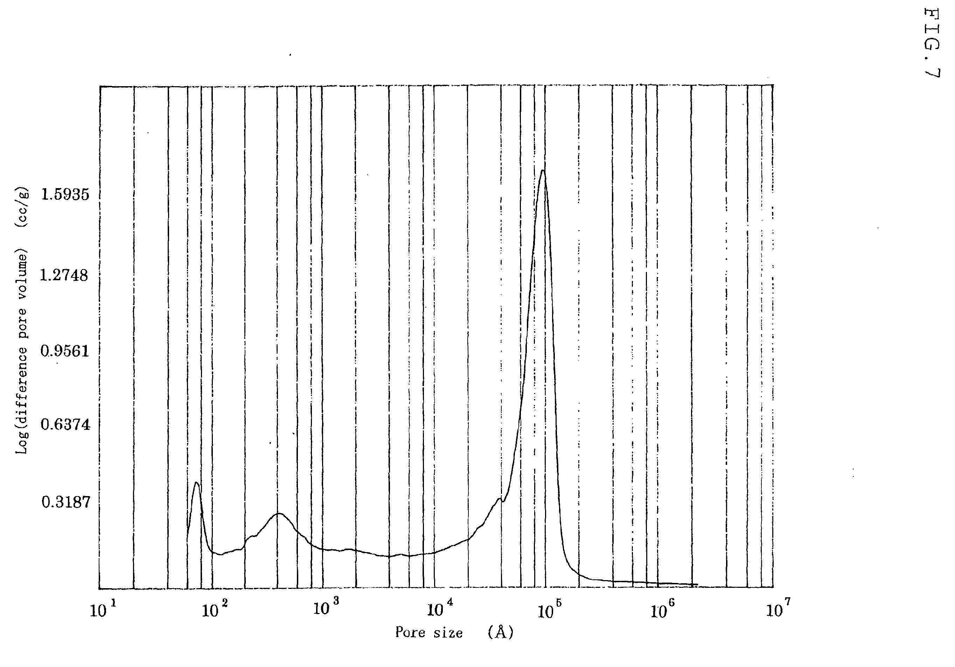

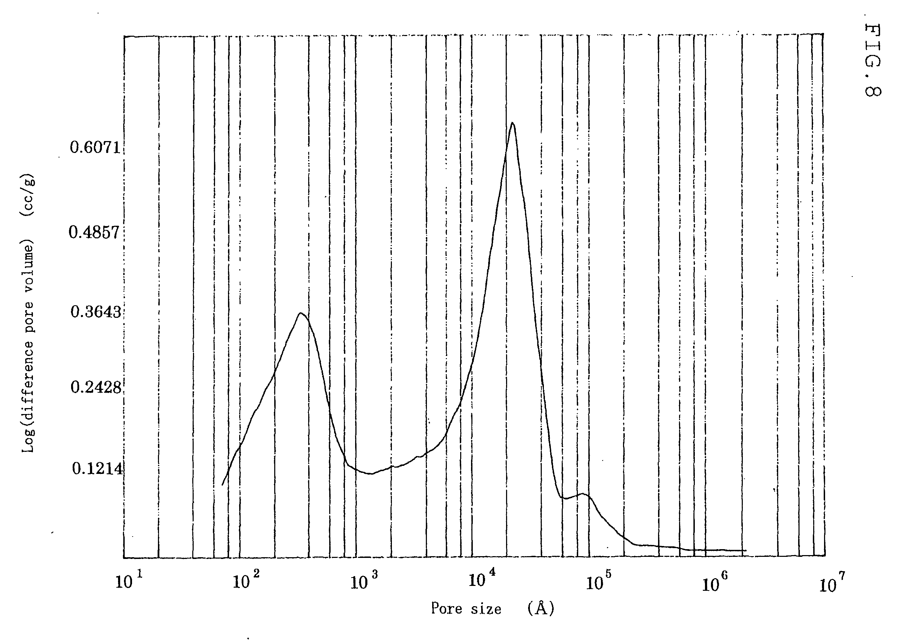

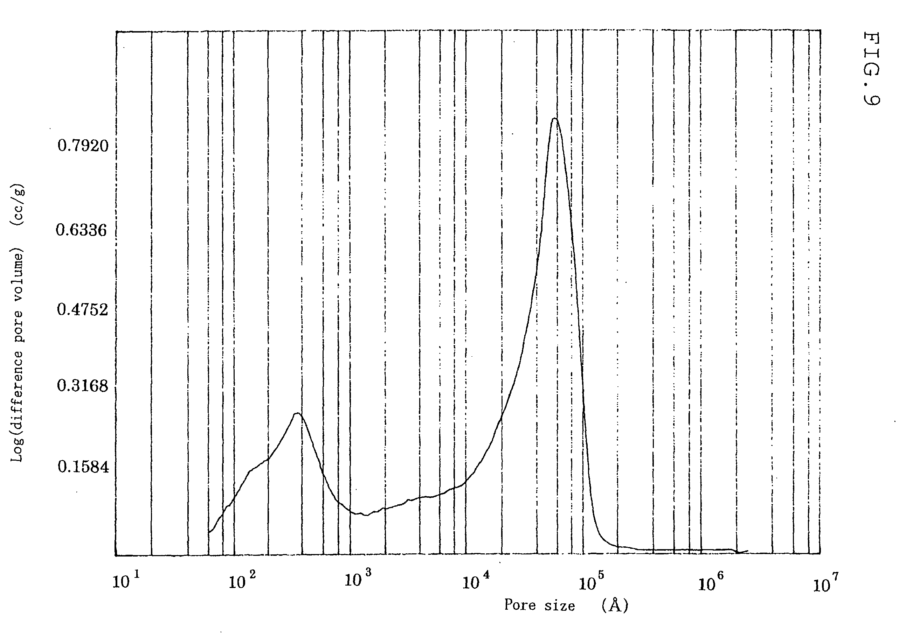

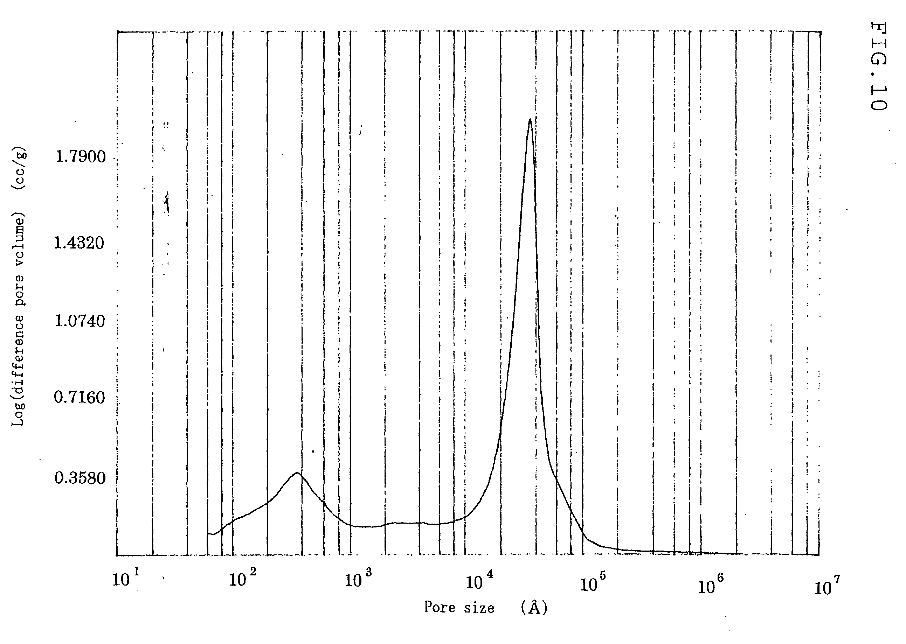

The pore size distribution profile of Ex. 1 sample containing colloidal silica at a solid content of 19.7%, that of Ex. 6b sample containing alumina cement at a solid content of 16.4%, and that of Ex. 6c sample containing alumina cement at a solid content of 28.2% were obtained through the mercury penetration method, and these profiles were compared with the profile of Comparative Example 1. The pore size distribution profiles are shown in Figs. 7 to 10. Porosity (%) values derived from these profiles are shown in Table 5.
The results indicate that the deodorizing articles of Examples 1, 6b, and 6c and Comparative Example 1 have a pore size peak falling within a range of 1 $\mu$m to 10 $\mu$m and a porosity corresponding to each distribution profile. Thus, the deodorizing articles of the Examples were confirmed to have an enhanced mechanical strength while deodorization performance is maintained.

[Table 5]

|  | Porosity (%) |
| --- | --- |
| Example 1 | 65.8 |
| Example 6b | 60.0 |
| Example 6c | 59.5 |
| Comparative Example 1 | 56.9 |

Industrial Applicability

As described hereinabove, by virtue of synergistic effect exerted by gypsum series and a clay mineral having meso-pores such as sepiolite or synergistic effect exerted through addition of a mineral having macro-pores of a pore size greater than that of the gypsum series (e.g., perlite), the deodorizing article of the present invention adsorbs an odor substance to thereby lower the odor level to a certain level or lower, when the target gas contains a large amount of an odor component, and releases the odor component without elevating the level of the released odor component to a certain level or higher, when the odor component level is lowered. By repeating the procedure, the odor level can be averaged and lowered to a certain level or lower, with the level being suppressed semi-permanently. In addition, by virtue of the white inorganic filler incorporated in the article, a mechanical strength such that the deodorizing article cannot readily collapsed during routine operation is attained.

**Claims**

1. A deodorizing article, **characterized in that** the deodorizing article is produced by dispersing in a medium a clay mineral having meso-pores, gypsum series, a mineral having a pore size greater than that of the gypsum series, and a white inorganic filler, and molding the formed dispersion.

2. A deodorizing article according to claim 1, which further contains a finely divided powder of a clay mineral having meso-pores.

3. A deodorizing article according to claim 1 or 2, which has a bending strength of 0.49 MPa or higher.

4. A deodorizing article according to any of claims 1 to 3, wherein the clay mineral is at least one species selected from sepiolite and palygorskite, and the mineral having a pore size greater than that of the gypsum series is expanded perlite.

**5.** A deodorizing article according to any of claims 1 to 4, which contains the clay mineral in an amount of at least 30 wt.% as solid content, the gypsum series in an amount of at least 25 wt.% as solid content, and the mineral having a pore size greater than that of the gypsum series in an amount of 30 wt.% or less as solid content.

**6.** A deodorizing article of any of claims 1 to 5, wherein the white inorganic filler is at least one species selected from colloidal silica and cement.

**7.** A deodorizing article according to claim 6, wherein the white inorganic filler is colloidal silica and is contained in an amount of 10 to 20 wt.% as solid content.

**8.** A deodorizing article according to claim 6, wherein the white inorganic filler is cement and is contained in an amount of 14 to 37 wt.% as solid content.

**9.** A deodorizing article according to any of claims 1 to 8, which is molded through slip cast molding.

**10.** A deodorizing article according to any of claims 1 to 9, which is to be used in an exhaust system connected to a kitchen.

**11.** A deodorizing article according to claim 10, which has a hollow cylindrical or columnar form and has a plurality of flow lines penetrated in an axial direction.

**12.** A method for producing a deodorizing article, **characterized in that** the method comprises the steps of dispersing in water a clay mineral having meso-pores, gypsum series, a mineral having a pore size greater than that of the gypsum series, and a white inorganic filler, to thereby form a dispersion; charging the dispersion into a mold through slip casting, and drying the dispersion and releasing the dried product from the mold.

**13.** A method for producing a deodorizing article according to claim 12, wherein the step of dispersing the members in water to thereby form a dispersion further includes dispersing a finely divided powder of a clay mineral having meso-pores.

**14.** A method for producing a deodorizing article according to claim 12 or 13, which further includes a step of preliminary firing the clay mineral having meso-pores at a predetermined temperature.

**15.** A method for producing a deodorizing article according to any of claims 12 to 14, wherein the clay mineral is at least one species selected from sepiolite and palygorskite, and the mineral having a pore size greater than that of the gypsum series is expanded perlite.

**16.** A method for producing a deodorizing article according to any of claims 12 to 15, wherein the deodorizing article contains the clay mineral in an amount of at least 30 wt.% as solid content, the gypsum series in an amount of at least 25 wt.% as solid content, and the mineral having a pore size greater than that of the gypsum series in an amount of 30 wt.% or less as solid content.

**17.** A method for producing a deodorizing article according to any of claims 12 to 16, wherein the white inorganic filler is at least one species selected from colloidal silica and cement.

**18.** A method for producing a deodorizing article according to claim 17, wherein the white inorganic filler is colloidal silica and is contained in an amount of 10 to 20 wt.% as solid content.

**19.** A method for producing a deodorizing article according to claim 17, wherein the white inorganic filler is cement and is contained in an amount of 14 to 37 wt.% as solid content.

FIG.1

FIG.2

Addition of Silicadol 20A

EP 1 473 045 A1

FIG.3

Addition of alumina cement

Exponential

Example 6e

Exponential

Example 6d

Exponential

Example 6c

Time elapsed after mixing (min)

FIG.4

(a)

(b)

(c)

FIG.5

Repetition times (times)

Percent adsorption performance
with respect to initial adsorption (%)

Ex. 6b-2

Ex. 6b-1

Ex. 6b

Adsorption-desorption repetition test

20

FIG.6

Adsorption-desorption test

FIG.7

FIG.8

FIG.9

FIG.10

# INTERNATIONAL SEARCH REPORT

| | International application No. |
|---|---|
| | PCT/JP03/00886 |

| A. | CLASSIFICATION OF SUBJECT MATTER |
|---|---|

Int.Cl$^7$ A61L9/014, 9/16, B01J20/12, B01D53/44

According to International Patent Classification (IPC) or to both national classification and IPC

| B. | FIELDS SEARCHED |
|---|---|

Minimum documentation searched (classification system followed by classification symbols)

Int.Cl$^7$ A61L9/014, 9/16, B01J20/12, B01D53/44

Documentation searched other than minimum documentation to the extent that such documents are included in the fields searched

| Jitsuyo Shinan Koho | 1926–1996 | Toroku Jitsuyo Shinan Koho | 1994–2003 |
|---|---|---|---|
| Kokai Jitsuyo Shinan Koho | 1971–2003 | Jitsuyo Shinan Toroku Koho | 1996–2003 |

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X<br>Y | JP 63-108083 A (Toyota Central Research And Development Laboratories, Inc., Toyota Motor Corp.),<br>12 May, 1988 (12.05.88),<br>Full text<br>(Family: none) | 1-10,12-19<br>11 |
| Y | JP 11-35381 A (Mizusawa Industrial Chemicals, Ltd.),<br>09 February, 1999 (09.02.99),<br>Full text; Fig. 5<br>(Family: none) | 11 |

☐ Further documents are listed in the continuation of Box C.    ☐ See patent family annex.

| * | Special categories of cited documents: |
|---|---|
| "A" | document defining the general state of the art which is not considered to be of particular relevance |
| "E" | earlier document but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 06 May, 2003 (06.05.03) | 20 May, 2003 (20.05.03) |

| Name and mailing address of the ISA/ | Authorized officer |
|---|---|
| Japanese Patent Office | |
| Facsimile No. | Telephone No. |

Form PCT/ISA/210 (second sheet) (July 1998)